# EUROPEAN PATENT APPLICATION

(11) **EP 2 280 063 A1**
(43) Date of publication of application: **02.02.2011**
(21) Application number: 09166640.4
(22) Date of filing: 28.07.2009
(51) Int. Cl.: C12N 1/16, C12N 15/62, C12P 21/02, C07K 14/415, C07K 16/28

(54) **Pichia pastoris as a host for the production of the ribosome-inactivating protein (RIP) saporin and saporin fusion chimaeras**

(71) Applicant: Consiglio Nazionale delle Ricerche, 00185 Roma (IT); Leukaemia Busters, Southampton, Hampshire SO16 6YD (GB); Universita' Degli Studi Di L'Aquila, 67100 L'Aquila (IT)
(72) Inventor: Ceriotti, Aldo, 00185 Roma (IT); Della Cristina, Pietro, 00185 Roma (IT); Colombatti, Marco, 00185 Roma (IT); Fabbrini, M. Serena, 00185 Roma (IT); Flavell, Sopsamorn U., Southampton, Hampshire SO16 6YD (GB); Flavell, David J., Southampton, Hampshire SO16 6YD (GB); Ippoliti, Rodolfo, Università degli Studi Aquila, 67010 L'Aquila (IT); Lombardi, Alessio, 00185 Roma (IT)
(74) Representative: Minoja, Fabrizio

(57) **Abstract**

The present invention relates to the field of recombinant protein expression and discloses a procedure for the production of toxic chimaeric proteins endowed with ribosome-inactivating activity based on the plant toxin Saporin from *Saponaria officinalis* in the methylotrophic yeast *Pichia pastoris*.

SEQUENCE LISTING

<110>  Consiglio Nazionale delle Ricerche
       Leukaemia Busters
       Università degli Studi dell'Aquila

<120>  PICHIA PASTORIS AS A HOST FOR THE PRODUCTION OF THE
       RIBOSOME-INACTIVATING PROTEIN (RIP) SAPORIN AND SAPORIN FUSION
       CHIMAERAS

<130>  2173EUR

<160>  7

<170>  PatentIn version 3.3

<210>  1
<211>  900
<212>  DNA
<213>  Saponaria officinalis

<400>  1
gaattcacga tgaaaatcta tgttgttgct actattgctt ggattttgtt gcaattttcc      60

gcttggacca ctactgatgc tgttacctcc attactttgg atttggttaa tcctactgct     120

ggtcaatact cctctttcgt tgataagatt agaaacaacg ttaaagaccc taatttgaag     180

tacggtggaa ctgacattgc tgttattgga ccaccatcta aagagaagtt tttgagaatt     240

aactttcaat cttccagagg tactgtttcc ttgggtttga agagagacaa cttgtacgtt     300

gttgcttact tggctatgga taacactaat gttaatagag cttactactt tagatccgaa     360

atcacctctg ctgagttgac tgctttgttc ccagaagcta ctacagctaa tcaaaaggct     420

ttggaatata ctgaggatta ccagtccatt gaaaagaatg ctcaaattac tcaaggagat     480

aagtccagaa aggagttggg tttgggaatc gacttgttgt tgacttctat ggaagctgtt     540

aataagaagg ctagagttgt taagaacgaa gctagattct tgttgattgc tattcaaatg     600

accgctgaag ttgctagatt cagatacatt caaaacttgg ttactaaaaa ttttcctaac     660

aagtttgatt ctgacaacaa ggttattcag ttcgaggttt cttggagaaa gatttctact     720

gctatctacg gagatgctaa gaacggagtt tttaacaagg attacgattt cggttttgga     780

aaagttagac aagttaagga cttgcaaatg ggtttgttga tgtatttggg aaagccaaag     840

tcttccaacg aggctaattc cactgcttac gctacaacag ttttgtaact cgaggatatc     900


<210>  2
<211>  425
<212>  DNA
<213>  Homo sapiens

<400>  2
ctcgagaaga gatctaatga attgcatcaa gttccatcca actgtgactg tttgaacgga      60

ggtacttgtg tttctaacaa gtacttttct aacattcatt ggtgtaactg tccaaagaag     120

tttggtggtc aacattgtga aattgacaaa tctaagactt gttacgaggg taatggtcat     180

ttttacagag gaaaggcttc tactgatact atgggtagac catgtttgcc atggaactct     240

gctactgttt tgcaacaaac ttaccatgct catagatccg atgctttgca attgggtttg     300

ggaaagcata actactgtag aaatccagat aacagaagaa gaccatggtg ttacgttcaa 360

gttggattga aaccattggt tcaagaatgt atggttcatg attgtgctga tggtaaagcg 420

gccgc 425

<210> 3
<211> 786
<212> DNA
<213> Homo sapiens

<400> 3
ggcccagccg gccatggctg aggttaaatt gatggaatcc ggtggtggtt tggttcaacc 60

aggtggatct atgaagttgt cctgtgttgc ttctggtttt actttttcca actactggat 120

gaactgggtt agacaatcac cagaaaaagg attggaatgg gttgctgaga ttagagttaa 180

atccaacaat tacgctactc actacgctga atctgttaga ggaagattca ctacctccag 240

agatgactcc aagtcttccg tttacttgca aatgaacaat ttgagaggtg aggatactgg 300

aatctactac tgcagtagag tttactacta tggtcacgac tacgctatgg attactgggg 360

tcaaggtacc tccgttactg tctcgagtgg ttccacttct ggttctggaa agccaggatc 420

aggagagggt tctaccaagg gatccgctgt tgacattgtt ttgactcaat ctccagctat 480

tatgtctact tcattgggtg aaagagttac tatgacttgt actgcttcat ctccagtctc 540

atctacctat ttgcactggt accaacaaaa gcctggttct tctcctaagt tgtggatcta 600

ctccacctct aagttggctt ccggtgttcc tgatagattt tctggatctg gttccggaac 660

ttcatactca ttgactattt cttccatgga agctgaggat gctgctacct actactgtca 720

ccagtaccac agatcaccaa gaaccttcgg tggtggtacc aaattggaga ttaaaagagc 780

ggccgc 786

<210> 4
<211> 28
<212> DNA
<213> Artificial

<220>
<223> synthetic oligonucleotide

<400> 4
ccggaattcg ttacctccat tactttgg 28

<210> 5
<211> 30
<212> DNA
<213> Artificial

<220>
<223> synthetic oligonucleotide

<400> 5
cagtagcggc cgcttacttt ggctttccca 30

<210> 6
<211> 29
<212> DNA

<213>  Artificial

<220>
<223>  synthetic oligonucleotide

<400>  6
cgctcgagaa aagaagcaat gaacttcat                                          29


<210>  7
<211>  25
<212>  DNA
<213>  Artificial

<220>
<223>  synthetic oligonucleotide

<400>  7
cagtagcggc cgcttttcca tctgc                                              25

## Description

The present invention relates to a process for the expression and secretion in the methylotrophic yeast *Pichia pastoris* of toxic chimaeric proteins having ribosome-inactivating activity, based on the plant ribosome-inactivating protein (RIP) saporin from soapworth *Saponaria officinalis.* The invention refers also to DNA sequences coding for a seed secretory saporin isoform.

### Background of the invention

Saporin belongs to the N-glycosidase family of plant toxins that includes the prototype RIP ricin: these are ribosome-inactivating proteins able to remove a specific adenine, found in an universally conserved "stem-loop" structure present in ribosomal RNAs from the large ribosome subunit (1, 2), leading to a block in protein synthesis and to apoptotic cell death in eukaryotic intoxicated cells.

The *Pichia pastoris* expression system makes use of a microbial eukaryotic host that can reach extremely high cell densities and has been widely used for high level expression of foreign secretory proteins which can be then purified from the extracellular culture media (3, 4). More recently, *P. pastoris* host cells have been successfully used for optimized expression of targeted toxins based on the diphtheria toxin (5).

Most of the targeting moieties such as antibody-fragments, growth factor domains or receptor ligands used to construct targeted toxins for anticancer therapy derive from secretory proteins. These normally fold in the oxidative environment of the endoplasmic reticulum (ER) and may require specific co-and post-translational modifications such as N-or O-glycosylation and disulphide bond formation to be produced in a proper conformation and in a biological active form. Therefore, their folding in bacterial cells can be quite inefficient, since bacterial host cells are devoid of the endomembrane system present in eukaryotic cells where newly synthesized polypeptides are assisted in their folding and undergo quality control mechanism(s) allowing the secretion of folded active proteins (4). In addition, the production of recombinant proteins in *E. coli* may have several disadvantages such as the formation of inclusion bodies containing heterologous protein needing to be refolded, and endotoxin contamination.

For instance, only low amounts of properly folded antimetastatic chimera made by the fusion of the Amino-Terminal Fragment of human urokinase (ATF) to the plant RIP saporin were recovered in bacterial host cells because of the presence in the ATF moiety of two disulfide-rich domains, a kringle and an EGF-like domain (6). ATF-saporin was instead secreted very efficiently when expressed in eukaryotic host cells protected from autointoxication by neutralizing antisaporin antibodies (7). This clearly shows that ER-targeting allows secretory ATF-saporin to reach micromolar concentrations in the extracellular medium, but also indicates that the production of these chimaeras in eukaryotic cells can be hampered by their intrinsic toxicity, due to the potential action of saporin towards the host ribosomes. In principle, because of the different sub-cellular localization of the newly synthesized recombinant polypeptide and the host ribosomes, this should not happen. On the other hand, efficiency of segregation of newly synthesized proteins in the endomembrane system can be far below 100% (8, 9) and mechanisms that can mediate the retrotranslocation to the cytosol are present in the endoplasmic reticulum (10). For all the above reasons, one may expect to observe some host toxicity during the expression of saporin polypeptides in an eukaryotic host cell. This may lead to a decrease in the biosynthetic ability of host cells. Under these suboptimal conditions, factors controlling translation efficiency may become crucial for the expression of the recombinant polypeptide.

### Description of the invention

The invention provides for optimized DNA gene sequences for expression of recombinant secretory saporin (sequence ID#1); of recombinant secretory ATF-saporin (sequence ID#2) and of recombinant secretory αCD22 fusion chimaeras (sequence ID#3).

The invention also provides a process for the preparation of a recombinant secretory saporin that comprises the steps of constructing a precursor saporin gene, inserting the precursor gene into *Pichia pastoris* GS115 *(his4)* host strain, followed by fermentation of the transformed clone to obtain secretory recombinant saporin.

Preferably, said precursor is constructed using pPICZalphaA vector and the cloning is carried out downstream AOX-1 promoter and Mating factor Alpha (MFalpha) signal sequence preserving the Glu-Ala repeats of Ste13 site and inserting a glutamic acid and a phenylalanine at the NH₂-terminus of mature saporin.

The invention also provides a process for the preparation of recombinant secretory ATF-saporin that comprises the steps of constructing an ATF-saporin precursor optimised gene, cloning into pPICSAPH vector, stably inserting the precursor gene into a *P. pastoris* host strain, followed by fermentation of the transformed clone to obtain secretory recombinant ATF-saporinH6.

The invention, in still a further embodiment, provides a process for the preparation of recombinant proteins endowed with anti-CD22 specificity containing a secretory single-chain variable fragment fused to saporin, comprising the steps of constructing a codon-optimised scFv precursor optimised sequence as a V_{H}-218L-V_{L} gene, that is then inserted in either pPICSAPH vector for single step purification of the fusion chimaera or in pITalphawt, and stably transforming *P. pastoris* host strain, followed by fermentation of the transformed clones to obtain purified secretory recombinant αCD22-saporin chimaeras.

The host is preferably ***Pichia pastoris* GS115 (his4) or SMD1168.**

### Detailed description of the invention

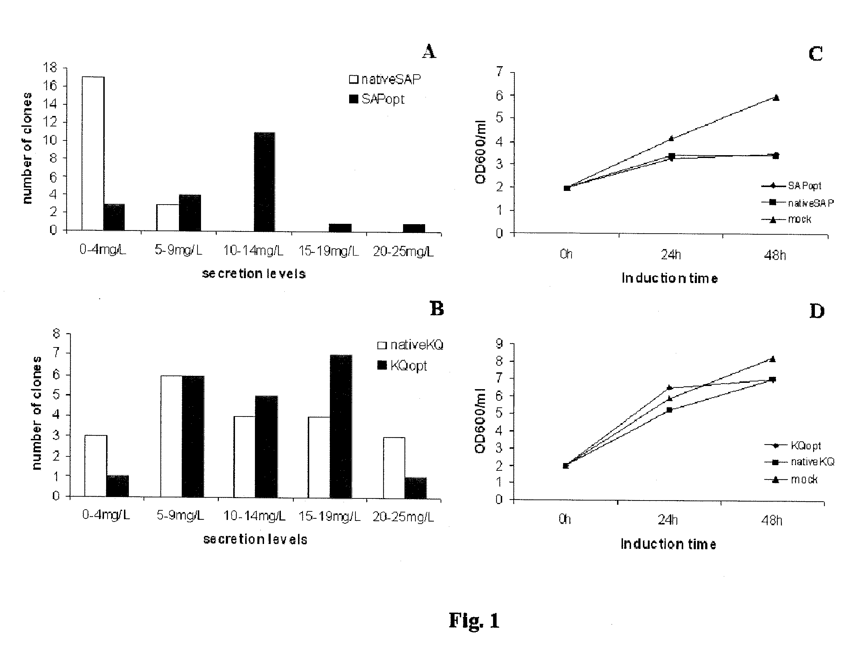

It has now been found a gene sequence (Sequence ID #1) allowing an efficient and convenient expression and production of saporin-based fusion proteins. The invention refers therefore to said sequence as well as to a process for the expression of proteins coded by said sequence in *Pichia pastoris* yeast strains. The process of the invention is improved as a consequence of increased levels of accumulation of the expressed protein **(****FIGURE 1****).**

The sequence of the invention has been obtained by codon-optimization for *Pichia* species usage (11, 5). The invention also provides the selection of GS115 *(his4)* as a host strain particularly tolerant to saporin expression (**FIGURE 2**), making also available expression vectors for producing untagged or hexahistidine-tagged saporin fusions (**FIGURES 3** **AND 4**) for easy purification of the fusion chimaeras.

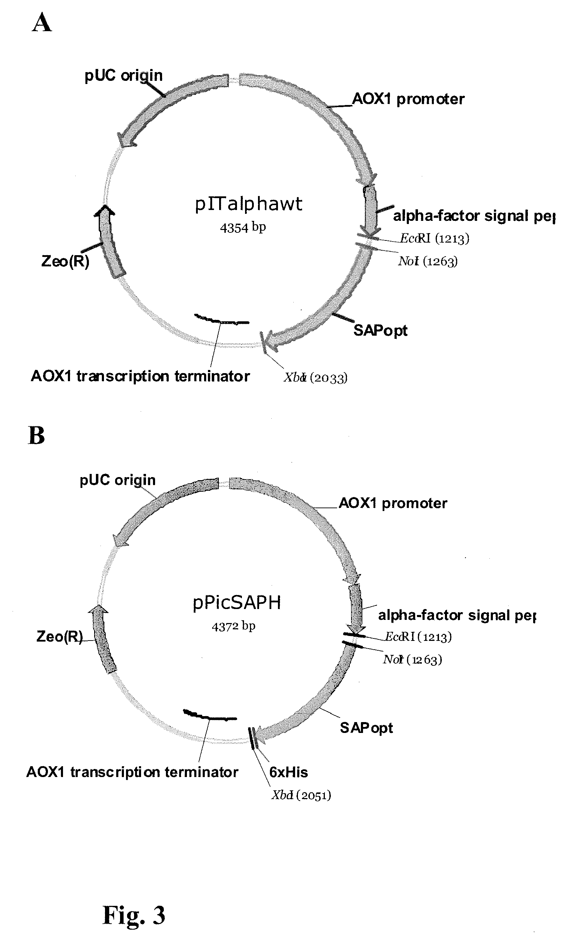

The present invention discloses novel recombinant strains allowing the production of an ATF-saporin chimaera in *P. pastoris* host cells in much larger amounts than previously achieved in bacterial host cells (6). The invention also provides two new scFv αCD22 fusion chimaeras, based on a single-chain variable fragment (scFv) obtained by RT-PCR of RNA extracted from a hybridoma producing a monoclonal antibody against the acute leukaemia marker antigen CD22 that was previously used to produce an anti-CD22 saporin immunotoxin (12). The sequence encoding the anti-CD22 scFv was first codon-optimized (Sequence ID #3) and then fused either to the codon optimized saporin sequence in pITalphawt vector (**FIGURE 3A**) for secretion of 4KBopt218LSAPopt fusion or to a sequence encoding hexahistidine-tagged saporin in pPICSAPH vector for secretion of 4KBopt218L-SAPHis6. The process of the invention is advantageous allowing increased yields of secretory recombinant proteins.

The DNA sequences coding for the toxic protein/chimaera are under the control of an inducible and tightly regulated promoter, such as the well known alcohol oxydase-1 promoter (AOX1) that can be repressed in the presence of dextrose/glucose during the biomass increase step and induced upon culturing the *P. pastoris* cells in the presence of methanol. Linearized DNAs are used to transform electrocompetent GS 115(*his4*) cells for integration of the expression cassette(s) into the host genome, ensuring stability of the recombinant transformed strain after repeated sub-culturing. The domain(s) used in the production of saporin chimaeras should impart good secretory properties and should also possibly undergo codon-optimisation.

The process of the invention preferably comprises the following steps:
1) Codon-optimization of the saporin cDNA sequence, based on the codon usage of *P. pastoris* (Sequence ID #1) and of the sequences for targeting domain(s) such as the Amino-terminal fragment of human urokinase (Sequence ID #2) and that coding for the single-chain variable fragment scFv αCD22 (Sequence ID #3).
2) Subcloning of said sequences in suitable recipient expression vectors for their integration into *P. pastoris* genome, preferably the pPICSAPH vector introducing a hexahistidine tag at the C-terminus of the saporin domain for metal-affinity purification of the secretory ATF-saporin chimaera or of the scFv αCD22 fusion termed 4KBopt218L-SAPHis6.
3) Transformation of electrocompetent GS115 *(his4)* host cells.
4) Selection of the best expressing clones in small-scale induction preparations by screening induced zeocine-resistant clones expressing secretory saporin, the histidine-tagged ATF-saporin or the fusion chimaeras possibly with the COOH-hexahistidine tag.
5) Medium-scale induction at 10 OD/mL in shake flask conditions of the most promising clone(s).
6) Concentration of the media for Nichel-affinity (IMAC) purification of the histidine-tagged fusion proteins or for cation-exchange purification of the secretory saporin and 4KBopt218L-SAPopt.
7) Assay for the cytotoxic activities of the purified recombinant protein(s) on leukaemia target cells.

In step 2, the toxic polypeptides should be directed to the extracellular space: suitable signal peptides for appropriate sub-cellular localization that must be placed at the NH2-terminus of the protein of interest include the signal sequences for targeting to the ER membrane (13) such as the plant signal peptide of saporin itself or as the signal and the propeptide region of the preproalpha mating factor domain of *Saccharomyces cerevisiae* (alpha-factor) which has been widely used for the expression of secretory proteins in *P. pastoris* (3,4) and is present in the pPICZalpha vectors commercially available from Invitrogen.

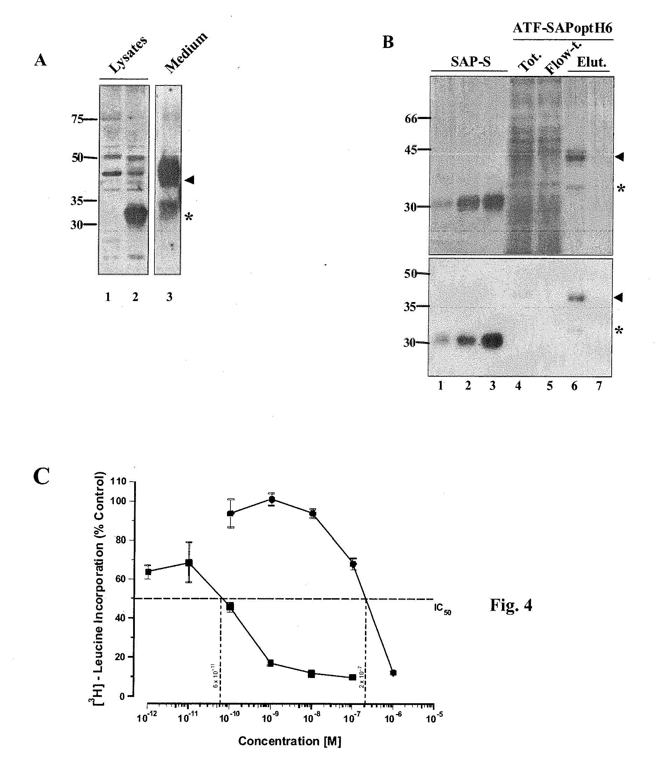

Different alternatives can be used in steps 1)-3) such as:
i) Use of different selected signal peptides of choice or the 90 amino acids of the alpha mating factor inserted at the NH2-terminus of the protein(s). The signal peptide of saporin was indeed effective in driving secretion of the mature protein in *P. pastoris.* When the alpha factor domain is used, it was found that the Ste13 aminodipeptidyl site(s) should be present, together with the Lys-Arg site recognized by the KEX-2 endoprotease, for proper processing by KEX-2 protease of the saporin precursor polypeptide.
ii) Use of the pPICSAPH vector inserting an hexahistidine-tag at the COOH-terminus of saporin allowing the expression of a secretory chimaera that can be purified by metal-affinity chromatography from the culture medium of the best expressing clone as in FIGURE 4 by using common IMAC procedures to obtain the tagged protein in a pure form.

The process of the invention is particularly advantageous for the expression of recombinant immunotoxins comprising a toxic domain having RIP activity (e.g. the saporin domain) fused to specific targeting domain(s) directing the fusion chimaera against selected tumor cells (14,15). Examples of said targeting domains comprise antibodies or single-chain antibody-fragments (scFv) against tumor antigens as in the case of 4KBopt218LSAPopt and 4KBopt218L-SAPHis6 fusions, or ligand binding domains, as in the case of the ATF domain binding to the human urokinase receptor, or growth-factor domains.

According to a preferred embodiment of this invention, the chimaeric fusion toxin ATF-saporin, targeting potentially metastatic tumor cells overexpressing the human urokinase-receptor, is produced in *P. pastoris* cells. ATF-saporinH6 consists of the amino-terminal fragment of human prourokinase (ATF), including the ligand-binding domain to the urokinase receptor, fused through a trialanine linker peptide to a saporin variant having 6 histidines at the C-terminus. A different chimaeric fusion between ATF and saporin (ATF-SAP) has been previously expressed with some difficulties in the cytosol *of E. coli* because of folding problems in the ATF domain, due to the presence of 6 disulphides (6). The RIP activity of the ATF-saporin chimaera produced in *Escherichia coli* or *Xenopus laevis* oocytes has been documented previously in details (6, 7). *Xenopus laevis* oocytes (7), are able to reproduce the co- and post-translational events during heterologous protein synthesis (16) but must be protected by co-injection of neutralizing anti-saporin antibodies, being sensitive to RIP activity (17, 18). *Xenopus* expression system has demonstrated to be a suitable model system to produce secretory ATF-saporin chimaera for analytical purposes but it is not a suitable expression system for the large scale expression needed for the clinical development of this class of molecules.

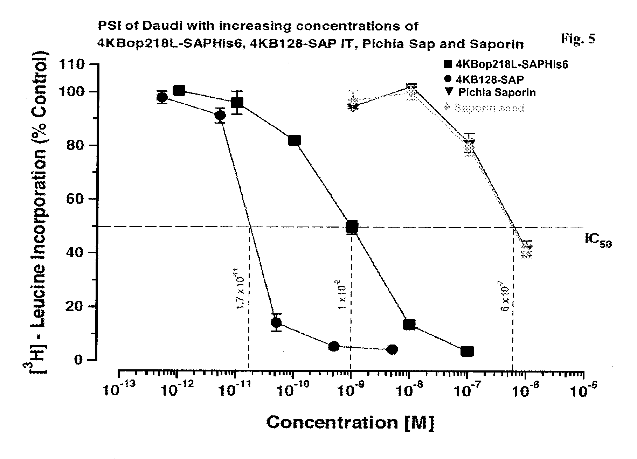

ATF-saporinH6 was secreted by GS115 *(his4)* cells at several mg/L and was easily purified in one step, as detailed in the following examples. The recombinant secretory chimaera shows the expected RIP activity against target cells, as also did the recombinant secretory saporin purified from the extracellular medium of a clone secreting up to 30 mg/L saporin in flask conditions, as described below. The scFvαCD22 fusion chimaera was also secreted at 2 mg/L in flask conditions whether having an hexahistidine-tag (4KBopt218L-SAPHis6) or not (4KBopt218LSAPopt) and the chimaeras were purified either by metal-affinity (4KBopt218L-SAPHis6) or by cation-exchange chromatography (4KBopt218LSAPopt). Importantly, both showed specific cytotoxic activity when assayed against target Audi cells (FIGURE 5) in a protein synthesis inhibition (PSI) assay.

### Description of the figures:

### Figure Legends:

### Figure 1: Effects of codon-optimization on saporin expression levels

*Pichia pastoris* GS 115 *(his4)* hosts cells were transformed with the native gene constructs or the codon-optimized constructs. (A-B) The histograms show the distribution of up to 20 independent GS115(*his4*) clones (y-axis, number of clones) induced 48 h for expression of either native (white) or optimized (opt; black) constructs coding for active saporin (A) or active-site mutant SAPKQ (B). The amount of secreted polypeptide was quantified by densitometry, using seed-extracted saporin as a reference standard. Expression levels (x-axis) are reported in different ranges in mg/L from the lowest (0-4mg/L) to the highest expressors (20-25mg/L). In the case of catalytically active saporin (A) clones expressing high levels of the protein were present only when the codon-optimized sequence (SAP_{OPT}) was used. Conversely, in the case of the active site mutant (B) clones accumulating high levels of the protein were present both when the native (nativeKQ) or the codon-optimized (KQ_{OPT}) sequences were used. (C-D) Growth curves of at least two mock-induced clones (triangles) for each experiment were compared to the averaged growth curves of 20 clones induced for expression of either the optimized (diamonds) or native saporin genes (squares) having an intact (C) or mutated catalytic site (D). Please note that independently from gene codon-optimization or the relative expression levels, when GS115(*his4*)cells synthesized active saporin polypeptides their growth rates decreased as compared to mock controls.

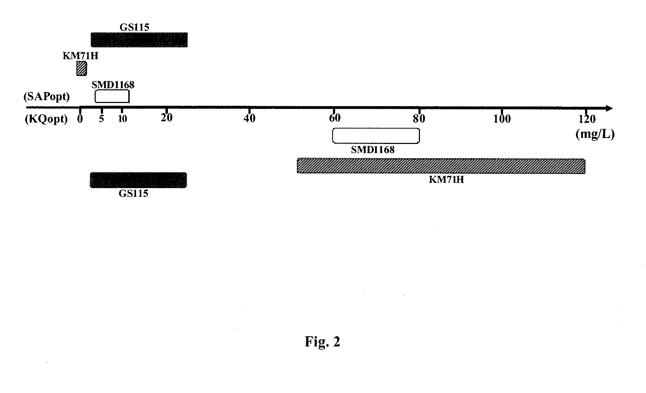

### Figure 2: Selection of the best suited host strains for saporin expression

14-20 randomly picked colonies expressing SAPopt or KQopt in three different hosts (KM71H, SMD1168, GS115 (*his4*)) were induced for 48h and the amount of saporin present in equivalent amounts of media was quantified by Western or slot-blots using seed-extracted saporin as a reference standard. The bars indicate the ranges of accumulation in the medium (mg/L) of either catalytically active (SAPopt, upper bars) or mutated (KQopt, lower bars) saporin in GS115 (*his4*) (black), KM71H (hatched) and SMD1168 (white) strains. Please note that only in GS 115 (*his4*) strain equivalent levels of secretion among active saporin and mutated KQ polypeptides could be obtained.

### Figure 3: Schematic diagram of the Fusion Expression Vectors pITalphawt (A) containing the codon-optimised SAPopt gene or (B) pPICSAPH containing the SAPopt gene with an extra hexahistidine tag (6xHis)

The vectors derive from pPicZalphaB vector (Invitrogen) and contains the pUC origin of replication, shown together with Alcohol oxydase-1 promoter (AOX1) and its transcription terminator, a zeocine resistance gene (ZeoR), the alpha mating factor signal peptide and prodomain, including the restriction enzyme sites present in the linker between *Eco*RI and *Not*I that is placed at 5' end of the SAPopt sequence: in this way a triple Alanine linker is inserted between any targeting domain and the plant toxin domain.

### Figure 4: Expression, single step purification of ATF-saporinH6 and determination of its cytotoxic activity

(A) Western with anti-saporin serum of 48 h medium-scale induction of the best GS 115 (*his4*) expressing clone producing ATF-saporinH6. Total cell lysate of cells expressing ATF-saporinH6 construct (lane 2) and with an equivalent amount of corresponding medium (lane 3) were analysed for comparison. Lane 1: mock-induced lysate as a control.

(B) The 48 h medium from induced cultures containing ATF-saporinH6 was concentrated and ATF-saporinH6 nichel-purified as described in Example 2 section. Total concentrated medium (Tot, lane 4), column flow-through (Flow-t., lane 5) and concentrated eluates (150 mM or 300 mM imidazole elution, lanes 6 and 7, respectively) were subjected to SDS-PAGE followed either by silver-staining (upper panel) or western-blotting with anti-saporin serum (lower panel). For quantifications, standard saporin (SAP-S) was loaded in lanes 1-3: 90, 225 and 450 ng (upper panel) or 30, 60 and 100 ng (lower panel). The position of ATF-saporinH6 is indicated by an arrowhead. The asterisk shows degradation products containing saporin-reactive polypeptides. Molecular weight markers positions are shown on the left. (C) ATF-saporinH6 activity was compared to that of saporin when assayed on acid-washed U937 human monocytes as described in (6, 7) and shows an IC₅₀ of 6 x 10⁻¹¹ M. Squares: ATF-saporinH6. Dots: seed-extracted saporin.

### Figure 5: Protein synthesis inhibition assay (PSI) on human leukaemia Daudi cells

4KBopt218L-SAPHis6 was produced and purified by Ni-affinity, essentially as described for ATF-saporinH6. Secretory saporin was purified by cation-exchange chromatography and human acute leukaemia Daudi cells were exposed as in (12) to increasing concentrations of purified histidine-tagged fusion (4KBop218L-SAPHis6, squares), recombinant secretory saporin purified from Pichia (Pichia Saporin, triangles), seed-extracted saporin (Saporin seed, diamonds) and to an anti CD22 saporin immunotoxin (4KB128-SAP IT, dots), as control, for 48h. At the end of the exposure the cells were washed and pulse-labelled with tritiated leucine essentially as described in (12), and results expressed as percent tritiated leucine incorporation relative to the untreated control Daudi cells. Please note that seed-extracted saporin and secretory saporin purified from *P. pastoris* medium have identical inhibitory activity showing an IC₅₀ of 6 x 10⁻⁷ M whereas the histidine-tagged fusion has the same cytotoxic activity as the untagged (not shown) being at least a hundred times more effective than non-targeted saporin against Daudi leukaemia cells, showing IC₅₀ of 1 x 10⁻⁹ M. A conventional saporin immunotoxin against CD22 leukaemia marker antigen, used as a control, showed an IC₅₀ of 1.7 x 10⁻¹¹ M.

The invention is disclosed in more detail in the following examples.

### EXAMPLE 1

### Construction of a vector for expression of secretory saporin in P. pastoris

A synthetic gene coding for a novel precursor of a seed-isoform of saporin (Sequence ID #1) whose DNA has been codon-optimized based on the yeast codon-usage reported among available *Pichia pastoris* coding sequences (CDS) in Biomed central [gbpln]: 111 CDS's (64359 codons) with triplet frequencies, choosing those most frequently represented in highly expressed *P. pastoris* proteins for the construction of this saporin synthetic gene which has been subcloned in pUC57 vector with EcoRI and EcoRV restriction sites (bold-face) was obtained as a pUC57SOL1GBXbaPst construct from GenScript Corporation, Piscataway, NJ.

Sequence ID #1:

1GAATTCACGATGAAAATCTATGTTGTTGCTACTATTGCTTGG
ATTTTGTTGCAATTTTCC61
GCTTGGACCACTACTGATGCTGTTACCTCCATTACTTTGGATTT
GGTTAATCCTACTGCT121
GGTCAATACTCCTCTTTCGTTGATAAGATTAGAAACAACGTTA
AAGACCCTAATTTGAAG181
TACGGTGGAACTGACATTGCTGTTATTGGACCACCATCTAAAG
AGAAGTTTTTGAGAATT241
AACTTTCAATCTTCCAGAGGTACTGTTTCCTTGGGTTTGAAGAG
AGACAACTTGTACGTT301
GTTGCTTACTTGGCTATGGATAACACTAATGTTAATAGAGCTT
ACTACTTTAGATCCGAA361
ATCACCTCTGCTGAGTTGACTGCTTTGTTCCCAGAAGCTACTAC
AGCTAATCAAAAGGCT421
TTGGAATATACTGAGGATTACCAGTCCATTGAAAAGAATGCTC
AAATTACTCAAGGAGAT481
AAGTCCAGAAAGGAGTTGGGTTTGGGAATCGACTTGTTGTTGA
CTTCTATGGAAGCTGTT541
AATAAGAAGGCTAGAGTTGTTAAGAACGAAGCTAGATTCTTGT
TGATTGCTATTCAAATG601

ACCGCTGAAGTTGCTAGATTCAGATACATTCAAAACTTGGTTA
CTAAAAATTTTCCTAAC661
AAGTTTGATTCTGACAACAAGGTTATTCAGTTCGAGGTTTCTTG
GAGAAAGATTTCTACT721
GCTATCTACGGAGATGCTAAGAACGGAGTTTTAACAAGGATT
ACGATTTCGGTTTTGGA781
AAAGTTAGACAAGTTAAGGACTTGCAAATGGGTTTGTTGATGT
ATTTGGGAAAGCCAAAG841
TCTTCCAACGAGGCTAATTCCACTGCTTACGCTACAACAGTTTT
GTAACTCGAGGATATC

The sequence coding for the mature protein was amplified using PFU-Taq DNA polymerase and the following oligonucleotides 5'CCGGAATTCGTTACCTCCATTACTTTGG-3' (sense oligo) (Sequence ID #4).

5'-CAGTAGCGGCCGCTTACTTTGGCTTTCCCA-3' (anti-sense oligo) (Sequence ID #5). The digested and purified *Eco*RI-*Not*I fragment was subcloned into *Eco*RI-*Not*I cut pPICZalphaA commercial vector to obtain SAPopt construct driving secretion of a mature saporin ending with Lys₂₅₃ and having, following Ste13 cleavage sites, an extra Glu and Phe (encoded within *Eco*RI site) at the N-terminus just before Val₁. To accurately evaluate host toxicity an equivalent construct was prepared in which two key residues at the catalytic site of saporin were mutated to produce a SAP**KQ** variant in which Glu₁₇₆ is changed into Lysine and Arg₁₇₉ into Glutamine and that has greatly reduced RIP activity (19). Codon-optimization advantageously affected yields **of** active saporin in the GS 115 (*his4*) strain (**FIGURES 1** **and** **2**). Three *Pichia pastoris* host strains have been further investigated and compared for the relative accumulation levels of the mutated SAP**KQ** and wild-type saporin (**FIGURE 2**) using the codon-optimized expression constructs. GS115(*his4*) was the most tolerant *P. pastoris* strain for the expression of saporin, since essentially no difference in the range of expression levels was found among clones expressing the SAPopt or the KQopt construct (and this strain was selected as the most promising host strain).

### EXAMPLE 2

### Construction of ATF-saporinH6 for secretion and single step purification of ATF-saporin chimaera

The aminoacid sequence 1-135 of human urokinase was amplified using as a template the plasmid pSP64TpAS (7) containing the human ATF DNA fragment, with a 5'-CGCTCGAGAAAAGAAGCAATGAACTTCAT-3' forward primer (Sequence ID #6) inserting the restriction enzyme site *Xho*I (underlined) and restoring the KEX-2 endoproteolytic Lys-Arg site just ahead the first aminoacid in ATF sequence (Ser₁) and a reverse primer 5'-CAGTAGCGGCCGCTTTTCCATCTGC-3' (Sequence ID #7) inserting a restriction enzyme site NotI (underlined) after Lys₁₃₅

The fragment encoding human ATF was digested with XhoI and NotI, purified and ligated to pPICSAPH vector opened with the same enzymes. Electrocompetent GS 115 *(his4)* cells were transformed and the best expressing clone selected following induction at 2 OD/mL with 0.5% Methanol. 10-25 µl of incubation medium after 48 h induction were assayed by slot-blot with an anti-saporin serum to identify the best expressing clones. Medium-scale preparation of the ATF-saporinH6 from this best expressing clone was achieved by growth 16h in liquid culture of a single freshly picked colony. For long term storage, cells (10-15 OD₆₀₀) were centrifuged 5 min at 1,560 g, resuspended in 1 ml of YPD [1% (w/v) yeast extract, 2% (w/v) peptone or tryptone, and 2% (w/v) dextrose] supplemented with 15% of sterile glycerol and frozen immediately. Stocks were stored at -80°C until use. For medium scale inductions, a freshly plated single colony obtained from these stocks was inoculated in 10 ml of YPD with 50 µg/mL Zeocin and 5 ml of this overnight preculture inoculated in a 2 L-flask containing 250 ml of the same medium, incubated at 30°C, 250 rpm overnigth. Cells were centrifuged at 1590g for 10 min at room temperature resuspended in 250 ml of BMMY [1% (w/v) yeast extract; 2% (w/v) peptone; 100mM phosphate buffer, pH 6.0; 1,34% (w/v) yeast nitrogen base, 4x10⁻⁵% (w/v) biotin; 4x10⁻³% (w/v) histidine; 0,5% (v/v) methanol] (without antibiotics) at final OD₆₀₀/ml of 10 and incubated at 30°C, 250 rpm for 48h. Methanol 0, 5% (v/v) and histidine 0, 04% final were added at the beginning of the induction period and 24 h later. At the end of 48 h induction period, cells were removed by centrifugation at 5000 g for 10 min at 4°C. The medium was exchanged against PBS pH 7.6 [137mM NaCl; 2,7mM KCl; 10mM Na₂HPO₄; 2mM KH₂PO₄] and concentrated 8-10 fold through a Hydrosart® Vivaflow 200 membrane, 10000 cut-off (Vivascience, Sartorius, Stedim Biotech, Firenze, Italy). The concentrated media containing the recombinant fusion proteins were supplemented with Complete™ or in the case of ATF-saporinH6 and 4kBopt218L-SAPHis6 with EDTA-free Complete™ (Roche, Monza, Italy), and then media were kept frozen before thawing at 30°C for subsequent protein purifications. ATF-saporinH6 protein was purified from the concentrated medium with IMAC nichel affinity and eluted as a single peak at 150 mM imidazole (**FIGURE 4**). Eluates were exchanged against PBS pH 7.6 and concentrated to 1 ml with Vivaspin columns 10000 cut-off concentrators (Vivascience, Sartorius, Stedim Biotech, Firenze, Italy) by centrifuging at 5000 g. Samples were analysed by SDS-PAGE and subjected to silver staining and seed-extracted saporin (SAP-S) was used as a reference standard. ATF-saporinH6 cytotoxic activity was assayed as described (6, 7). SAP-S was purified from *Saponaria officinalis* seeds as previously described (20).

As a further improvement of the above described procedure, the sequence coding for the ATF domain was optimized for expression in *Pichia pastoris.* A codon-optimised ATF fragment (Sequence ID # 2) with codon-usage of *Pichia pastoris* species was designed following the same rules described for the saporin codon-optimised sequence. The resulting synthetic DNA (sequence ID#2) was obtained from GenScript Corporation, Piscataway, NJ subcloned in pUC57 with EcoRI and EcoRV restriction sites to obtain pUC57ATFopt construct.

Sequence ID #2: ATF optimised gene sequence (XhoI-NotI fragment, restriction sites in bold) is shown below:

CTCGAGAAGAGATCTAATGAATTGCATCAAGTTCCATCCAACT
GTGACTGTTTGAACGGAGGTACTTGTGTTTCTAACAAGTACTTTTCTA
ACATTCATTGGTGTAACTGTCCAAAGAAGTTTGGTGGTCAACATTGT
GAAATTGACAAATCTAAGACTTGTTACGAGGGTAATGGTCATTTTTA
CAGAGGAAAGGCTTCTACTGATACTATGGGTAGACCATGTTTGCCAT
GGAACTCTGCTACTGTTTTGCAACAAACTTACCATGCTCATAGATCC
GATGCTTTGCAATTGGGTTTGGGAAAGCATAACTACTGTAGAAATCC
AGATAACAGAAGAAGACCATGGTGTTACGTTCAAGTTGGATTGAAA
CCATTGGTTCAAGAATGTATGGTTCATGATTGTGCTGATGGTAAAGC
GGCCGC

### EXAMPLE 3

### Construction of the scFv αCD22 fusion 4KBopt218L-SAPopt or the histidine-tagged 4KBopt218L-SAPHis6 for metal-affinity purification

The single-chain variable sequences were obtained with standard procedures by reverse transcription-PCR on RNA obtained from the hybridoma producing anti CD22 monoclonal antibody that was used for producing an anti-CD22 SAP immunotoxin (12). A synthetic codon-optimized DNA was obtained from GenScript Corporation, Piscataway, NJ and DNA subcloned in pUC57 with EcoRI-HindIII to obtain pUC57αscFvCD22opt construct and including SfiI and NotI restriction sites for subcloning into pITalphawt or pPICSAPH. Sequence ID #3 is shown below and corresponds to the anti-CD22 optimised sequence including a flexible linker joining the V_{H} and V_{L}, known as 218 linker (21), in the V_{H}-218L-V_{L} configuration. SfiI and NotI restriction sites are shown in bold.

GGCCCAGCCGGCCATGGCTGAGGTTAAATTGATGGAATCCGG
TGGTGGTTTGGTTCAACCAGGTGGATCTATGAAGTTGTCCTGTGTTG
CTTCTGGTTTTACTTTTTCCAACTACTGGATGAACTGGGTTAGACAAT
CACCAGAAAAAGGATTGGAATGGGTTGCTGAGATTAGAGTTAAATC
CAACAATTACGCTACTCACTACGCTGAATCTGTTAGAGGAAGATTCA
CTACCTCCAGAGATGACTCCAAGTCTTCCGTTTACTTGCAAATGAAC
AATTTGAGAGGTGAGGATACTGGAATCTACTACTGCAGTAGAGTTTA
CTACTATGGTCACGACTACGCTATGGATTACTGGGGTCAAGGTACCT
CCGTTACTGTCTCGAGTGGTTCCACTTCTGGTTCTGGAAAGCCAGGA
TCAGGAGAGGGTTCTACCAAGGGATCCGCTGTTGACATTGTTTTGAC
TCAATCTCCAGCTATTATGTCTACTTCATTGGGTGAAAGAGTTACTAT
GACTTGTACTGCTTCATCTCCAGTCTCATCTACCTATTTGCACTGGTA
CCAACAAAAGCCTGGTTCTTCTCCTAAGTTGTGGATCTACTCCACCTC
TAAGTTGGCTTCCGGTGTTCCTGATAGATTTTCTGGATCTGGTTCCGG
AACTTCATACTCATTGACTATTTCTTCCATGGAAGCTGAGGATGCTG
CTACCTACTACTGTCACCAGTACCACAGATCACCAAGAACCTTCGGT
GGTGGTACCAAATTGGAGATTAAAAGAGCGGCCGC.

This DNA was subcloned using the SfiI-NotI sites (in bold) into pPICSAPH opened with the same restriction enzymes to obtain 4KBopt218L-SAPHis6 or into the vector pITalphawt containing SAPopt to obtain 4KBopt218L-SAPopt as an identical construct except for the absence of the C-terminal 6 histidines. The same procedure as described for ATF-saporinH6 was followed to obtain and analyze the induced clones expressing the scFv-saporin fusions and to select the best expressing clones to be induced at 10 OD/mL in flask conditions, except that induction was performed at 23°C. At the end of the 48 h induction period the media were concentrated exactly as described for ATF-saporinH6 and subjected either to cation-exchange chromatography (4KBopt218L-SAPopt) or to nichel-affinity chromatography (4KBopt218L-SAPHis6), in the same conditions as reported above for ATF-saporinH6. The purified proteins were assayed against target leukaemia Daudi cells and their cytotoxicities compared, showing no significant differences when estimated as the concentration inhibiting 50% incorporation of tritiated leucine relative to control untreated Daudi cells (IC50). **FIGURE 5** shows the dose-response curves obtained by exposing Daudi cells to 4KBopt218L-SAPHis6 or saporin secreted by *P. pastoris,* to seed-extracted saporin and to the anti-CD22 immunotoxin (12).

### References

1. Soria, M. R., et al. (1992) Targeted Diagn. Ther. 7, 193-21.
2. Peumans, W. J., et al. (2001) FASEB J. 15, 1493-1506.
3. Cereghino, J. L., et al. (2000) FEMS Microbiol. Rev. 24, 45-66.
4. Daly, R.., and Hearn, M. T. (2005) J. Mol. Recognit. 18, 119-38.
5. Woo, J. H., et al. (2002) Protein Expr. Purif. 25,270-282.
6. Fabbrini, M. S., et al. (1997) FASEB J. 11, 1169-1176.
7. Fabbrini, M. S., et al. (2000) FASEB J. 14, 391-398.
8. Hegde, R. S., et al. (2006) Trends Biochem. Sci. 31,563-71.
9. Shaffer, K. L., et al. (2005) Dev. Cell 9,545-554.
10. Plemper, R. K., et al. (1999) Trends Biochem. Sci. 24, 266-70.
11. Sreekrishna, K. (1993) In Industrial Microorganism: Basic and Applied Molecular Genetics, Am. Soc. Microbiol. (Baltz, R.H., Hegeman,G.D. and Skatrud, P.L., eds) pp.119-126, Washington DC.
12. Flavell, D. J., et al. (1997) Cancer Res. 7, 4824-4829.
13. Rapoport, T. A. (1991) FASEB J. 5, 2792-8.
14. Fabbrini, M. S., et al. (2003) in "Bacterial, Plant&Animal Toxins" (Ascenzi, P., Polticelli, F. a., and Visca, P., Eds.), pp. 69-99, Research Signpost, Kerala, India.
15. Flavell, D. J. (1998) Curr. Top. Microbiol. Immunol. 234, 57-61.
16. Bossi, E., et al. Methods Mol. Biol. 375, 107-31. Review
17. Saxena, S. K., et al. (1989) J. Biol. Chem. 264, 596-601.
18. IT 1298437
19. Zarovni, N., et al. (2007) Cancer Gene Ther. 14, 165-73.
20. Flavell, D.J., et al. (2001) Br. J. Cancer. 84,571.
21. Rosemblum, M. G., et al. (2003) Cancer Res. 63, 3995-4002.

## Claims

1. A DNA sequence for expression of recombinant secretory saporin of sequence ID#1.

2. A DNA sequence for expression of recombinant secretory ATF-saporin of sequence ID#2.

3. A DNA sequence for expression of recombinant fusions **4KBopt218L-SAPopt** or **4KBopt218L-SAPHis6** of sequence ID#3.

4. A process for the preparation of a recombinant secretory saporin that comprises the steps of constructing a synthetic precursor saporin gene, inserting the precursor gene into *Pichia pastoris* GS115 *(his4)* host strain, followed by fermentation of the transformed clone to obtain secretory recombinant saporin.

5. The process as claimed in claim 3 wherein said precursor is constructed using pPICZalphaA vector.

6. The process as claimed in claim 5 wherein said cloning is carried out downstream AOX-1 promoter and Mating factor Alpha (MFalpha) signal sequence by keeping the Glu-Ala repeats of Ste13 site and inserting a Glutamic acid and a Phenylalanine at the NH₂-terminus of mature saporin.

7. A process for obtaining recombinant secretory ATF-saporin that comprises the steps of constructing a precursor ATF-saporin optimised gene by cloning the ATF-encoding sequence into the pPICSAPH vector, stably inserting the precursor gene into a *P. pastoris* host strain, followed by fermentation of the transformed clone to obtain secretory recombinant ATF-saporinH6.

8. The process as claimed in claim 7 wherein said cloning is carried out downstream AOX-1 promoter and Mating factor Alpha (MFalpha) signal sequence present in the pPICSAPH vector providing an hexahistidine-tag for single step purification of the fusion chimaera.

9. A process for the preparation of recombinant secretory single chain variable fragment fusions to saporin endowed with anti-CD22 specificity, comprising the steps of constructing the optimised V_{H}-218L-V_{L} gene, inserting the sequence in either pPICSAPH vector for single step purification of the fusion chimaera or as a fusion to untagged saporin in pITalphawt and stably transforming *P. pastoris* host strain, followed by fermentation of the transformed clone to obtain pure secretory recombinant αCD22-saporin chimaeras.

10. The process as claimed in claim 9 wherein said host is selected among *Pichia pastoris* hosts GS115 *(his4)* or SMD1168.

11. The process as claimed in claims 4, 7 and 9 wherein said fermentation is carried out in a suitable medium containing dextrose during the initial phase of fermentation to accumulate biomass.
